# DEMANDE DE BREVET EUROPEEN

(11) **EP 2 255 721 A1**
(43) Date de publication de la demande: **01.12.2010**
(21) Numéro de dépôt: 10163794.0
(22) Date de dépôt: 25.05.2010
(51) Int. Cl.: A61B 1/267

(54) **Laryngoscope équipé d'une caméra**

(30) Priorité: 27.05.2009 FR 0902553
(71) Demandeur: Dioptik, 87069 Limoges Cedex (FR)
(72) Inventeur: Faugeras, Pierre, 87000, Limoges (FR); Vincent, Jacques, 11100, Narbonne (FR)
(74) Mandataire: Myon, Gérard Jean-Pierre

(57) **Abrégé**

Ce laryngoscope comportant :
- une poignée formant un logement pour une caméra dont l'objectif est affleurant à une partie (8) de la poignée,
- une lame de laryngoscope (11) montée de manière amovible sur la poignée,
- un guide d'images (19) monté sur la lame (11) et adapté pour transmettre les images entre une extrémité de la lame et la caméra, **caractérisé en ce qu**'il comprend également :
- un premier moyen de connexion (23, 120), entre l'objectif de la caméra et une lentille optique (240) montée à une extrémité du guide d'images (19), adapté pour faire passer la lentille optique (240) d'une position où cette lentille (240) est éloignée de l'objectif (12) de la caméra, à une position où la lentille optique (240) est à proximité et alignée avec l'objectif (12) de la caméra, leurs axes optiques étant confondus,
- un deuxième moyen de connexion (15, 16) entre la lame (11) et la poignée (2), adapté pour faire passer la lame (11) d'une position où elle n'est pas bloquée en configuration d'utilisation, sur la poignée (2), à une position où la lame (11) est bloquée en configuration d'utilisation sur la poignée et en ce que le passage (F₁) de la lame (11) à la position où elle est en configuration d'utilisation entraîne le passage (F₁) de l'extrémité (22) du guide d'images (19) équipée de la lentille optique (240) dans la position où cette lentille (240) est à proximité et alignée avec l'objectif (12) de la caméra.

## Description

L'invention a trait à un laryngoscope équipé d'une caméra.

Un laryngoscope est un instrument médical utilisé, lors d'une anesthésie, pour maintenir la langue d'un patient lors de l'introduction d'une sonde d'intubation dans la trachée du patient. Pour cela, le laryngoscope comprend une poignée sur laquelle est montée une lame courbe épousant, globalement, la forme de la cavité buccale et de la langue d'un patient, lorsque le laryngoscope est en position. Les dimensions et/ou la forme de la lame varient légèrement selon que le patient est un enfant ou un adulte. Pour des questions d'hygiène, la lame du laryngoscope doit être propre. Pour cela, il est fréquent d'utiliser des lames de laryngoscope à usage unique. De telles lames sont montées sur la poignée par un système de fixation amovible.

Afin de faciliter et d'améliorer la vision du praticien lors de la mise en place du laryngoscope, puis de la sonde d'intubation, les lames sont pourvues de différents accessoires tels qu'un dispositif d'éclairage, par exemple par fibre optique ou LED, ou d'un dispositif de visualisation. Ce dispositif de visualisation est, par exemple, une caméra située à l'extrémité de la lame et reliée à un écran déporté.

On connaît, par US-A-2007/0179342, un laryngoscope dont la lame est équipée d'une caméra reliée à la poignée du laryngoscope. Cette poignée abrite une source d'énergie ainsi qu'une antenne permettant une liaison sans fil avec un écran distant. Une telle solution implique, soit d'avoir un laryngoscope complètement stérilisable, soit de réaliser des lames à usage unique avec un prix unitaire relativement élevé puisqu'elles abritent une caméra. On connaît également par US-A-2005/0148821 un laryngoscope dont la lame, amovible et à usage unique, est, en position de service, fixée sur une poignée dans laquelle est logée une caméra. La poignée est reliée par un câble à un écran. Un guide d'images est fixé sur la lame et permet d'acheminer les images jusqu'à la caméra, à partir de l'extrémité de la lame. Le guide d'images comprend une fibre optique insérée dans des orifices de maintien et de guidage ménagés sur la lame. Ce guide d'images est amovible par rapport à la lame et une de ses extrémités dépasse de la lame. Cette extrémité est reliée, par un embout, à un logement situé sur la poignée et dans lequel se trouve l'objectif de la caméra. Si un tel dispositif permet effectivement l'utilisation d'une lame à usage unique sur un laryngoscope muni d'une caméra, la mise en place du guide d'images sur la lame puis la fixation de son extrémité dans le logement abritant la caméra ne sont pas aisés.

US-A-2002/087050 décrit un laryngoscope dont la poignée est pourvue d'un logement pour un dispositif de capture d'images. La mise en place de la lame sur la poignée s'effectue soit par une liaison de type baïonnette soit par un mouvement en arc de cercle. US-A-2005/059863 concerne un laryngoscope dont la poignée reçoit une caméra et un écran.

Dans ces deux documents, le montage des différentes pièces du laryngoscope est relativement long et délicat, sans compter les risques d'usure et/ou de détérioration au niveau de la zone de connexion entre l'extrémité du guide d'images et la poignée. Par ailleurs le trajet des images via le guide d'images entre la lame et la caméra insérée dans la poignée est relativement complexe.

C'est à ces inconvénients qu'entend plus particulièrement remédier l'invention en proposant un laryngoscope équipé d'une caméra, utilisable avec des lames à usage unique, d'un faible de coût de production et assurant une connexion simple des différents éléments constitutifs du laryngoscope.

A cet effet, l'invention a pour objet un laryngoscope comportant :
- une poignée formant un logement pour une caméra dont l'objectif est affleurant à une partie de la poignée,
- une lame de laryngoscope montée de manière amovible sur la poignée,
- un guide d'images monté sur la lame et adapté pour transmettre les images entre une extrémité de la lame et la caméra, **caractérisé en ce qu**'il comprend également :
- un premier moyen de connexion, entre l'objectif de la caméra et une lentille optique montée à une extrémité du guide d'images, adapté pour faire passer la lentille optique d'une position où cette lentille est éloignée de l'objectif de la caméra à une position où la lentille optique est à proximité et alignée avec l'objectif de la caméra, leurs axes optiques étant confondus et situés dans le prolongement d'un axe principal de la lame,
- un deuxième moyen de connexion entre la lame et la poignée, adapté pour faire passer la lame d'une position où elle n'est pas bloquée en configuration d'utilisation, sur la poignée, à une position où la lame est bloquée en configuration d'utilisation sur la poignée et en ce que le passage de la lame à la position où elle est en configuration d'utilisation entraîne le passage de l'extrémité du guide d'images équipée de la lentille optique dans la position où cette lentille est à proximité et alignée avec l'objectif de la caméra.

Ainsi, la mise en position de la lame sur la poignée du laryngoscope s'accompagne également de la connexion entre l'objectif de la caméra et le guide d'images. Les opérations de montage en sont simplifiées et l'on réalise une mise en place optimale.

Selon des aspects avantageux mais non obligatoires de l'invention, un tel laryngoscope peut incorporer une ou plusieurs des caractéristiques suivantes :
- Le moyen de connexion comprend un logement, ménagé sur la poignée, et configuré en forme de coin tronqué à faces asymétriques.
- Le moyen de connexion comprend un organe configuré en forme de polyèdre tronqué, à faces asymétriques, et disposé à une extrémité du guide d'images.
- Le logement et le polyèdre, de formes complémentaires, sont respectivement positionnés pour que leurs parties tronquées reçoivent, d'une part, l'objectif de la caméra, et, d'autre part, la lentille optique de l'extrémité du guide d'images.
- Une des faces du logement et une des faces du polyèdre sont à courbure sphérique.
- Le passage de la lame à une configuration d'utilisation sur la poignée et le passage de l'extrémité du guide d'images dans la position où la lentille optique est à proximité de l'objectif de la caméra suivent des trajectoires en arc de cercle.
- La poignée est équipée d'un écran de visualisation.
- L'écran de visualisation est positionné selon l'axe principal de la lame.
- L'écran est déporté, la liaison entre l'écran et la poignée du laryngoscope étant filaire ou non.
- La lame est pourvue, à son extrémité adaptée pour être montée sur la poignée, d'un organe autorisant le montage seulement d'une lame de ce type sur cette poignée.

L'invention sera mieux comprise et d'autres avantages de celle-ci apparaîtront plus clairement à la lecture de la description qui va suivre d'un mode de réalisation d'un laryngoscope conforme à l'invention, donnée uniquement à titre d'exemple et faite en se référant aux dessins annexés dans lesquels :
- la figure 1 est une vue en perspective, de trois quart avant, d'un laryngoscope conforme à l'invention, en configuration de service,
- la figure 2 est une vue de côté du laryngoscope de la figure 1, à la même échelle, une partie de la lame étant arrachée afin de visualiser le guide d'images,
- la figure 3 est une vue similaire à la figure 1, le guide d'images et l'objectif de la caméra étant en position déconnectée, la lame étant montée sur la poignée,
- la figure 4 est une vue similaire à la figure 2, le guide d'images et l'objectif de la caméra étant en position déconnectée et la lame étant montée sur la poignée,
- la figure 5 est une vue en perspective, de trois quart avant, à la même échelle, de la poignée du laryngoscope seule, sans la lame,
- la figure 6 est une coupe selon le plan VI, à plus grande échelle, de la zone de montage de la lame sur la poignée, l'objectif de la caméra et le guide d'images étant en position déconnectée, la lame étant montée sur la poignée,
- la figure 7 est une vue de côté, à la même échelle que la figure 6, du guide d'images seul, celui-ci étant muni du dispositif de connexion,
- la figure 8 est une vue de face, à la même échelle que la figure 7, de l'extrémité du guide d'images pourvu du dispositif de connexion,

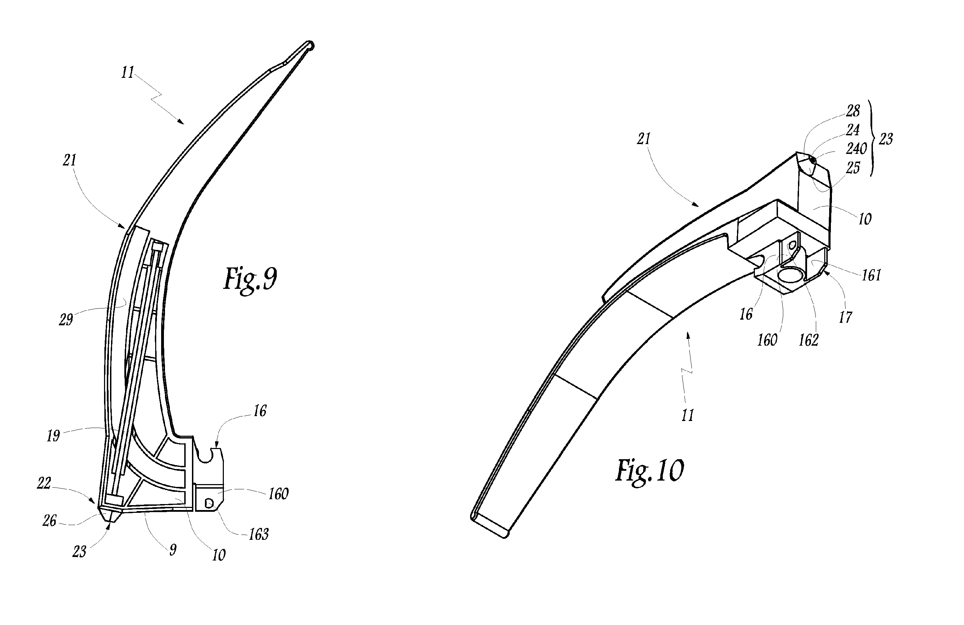

- la figure 9 est une vue de côté, à une autre échelle, de la lame seule, la partie abritant le guide d'images étant partiellement arrachée et
- la figure 10 est une vue de dessous, à la même échelle, de la lame de la figure 9, montrant le dispositif de montage de la lame sur la poignée.

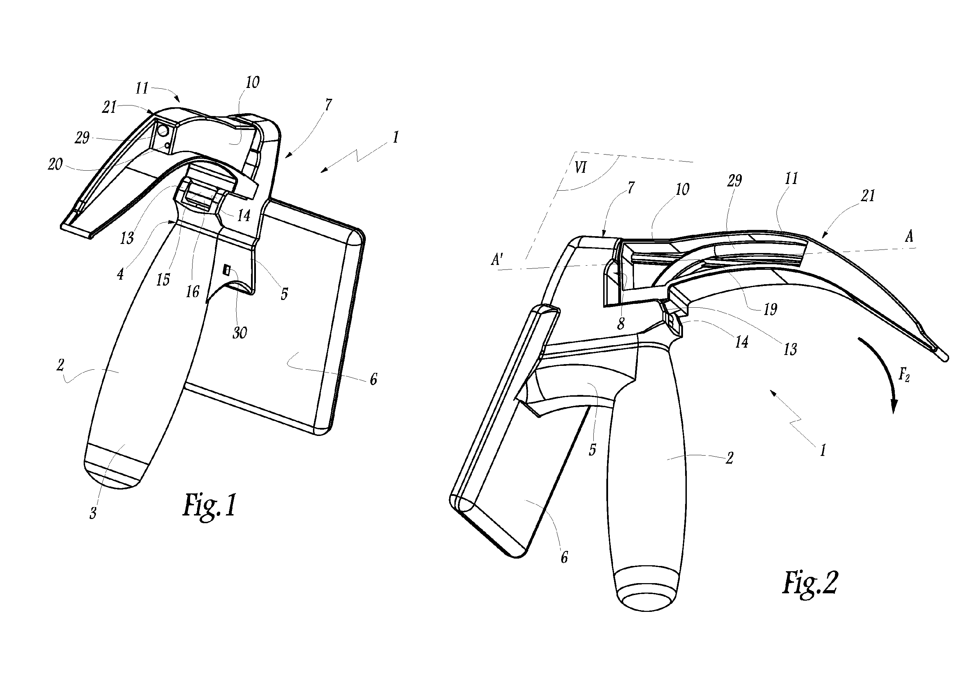

Le laryngoscope 1 représenté à la figure 1 comprend une lame 11 et une poignée 2. La poignée 2, de forme ergonomique, est réalisée en un matériau rigide aisé à nettoyer et inerte vis-à-vis des produits chimiques. Il s'agit, par exemple, d'un polymère ou d'acier inoxydable. Cette poignée 2 est équipée d'une extrémité libre 3 amovible, par exemple par dévissage, permettant l'accès à l'intérieur de la poignée. La poignée 2 abrite une source d'énergie, non illustrée, par exemple des piles ou une batterie rechargeable. Dans le cas d'une batterie rechargeable, la poignée 2 est pourvue de connecteurs ou contacts permettant la mise en place de la poignée sur un socle de rechargement, non illustré.

L'extrémité 4 opposée à l'extrémité 3 de la poignée 2 comprend une patte 5 sur laquelle est fixé un écran de visualisation 6. Cet écran de visualisation, connu en soi, est un écran 6 de type LCD ou de type OLED, rectangulaire et de dimensions voisines de 10 cm X 8 cm.

Dans l'exemple, l'écran 6 est monté fixe sur la patte 5. Il est disposé de manière à permettre une vision des cordes vocales tant sur l'écran qu'en direct. Pour cela, l'écran 6 est globalement perpendiculaire à un axe principal A-A' de la lame 11. En variante, il est orientable. Par exemple, il peut pivoter, de plus ou moins 90° à partir de cette position afin de se rapprocher ou de s'éloigner de la poignée.

L'extrémité 4 de la poignée 2, au dessus de la patte 5, comprend une pièce 7 désignée par la suite par le terme de tête. Cette tête 7 abrite une caméra, non représentée, celle-ci étant connue en soi. La caméra est alimentée par la source d'énergie contenue dans la poignée 2. L'objectif de la caméra est situé sur une face 8 de la tête 7. Cette face 8 est orientée en direction du patient lorsque le laryngoscope 1 est en service. En d'autres termes, cette face 8 est celle située en regard d'une face 9 d'une extrémité ou talon 10 de la lame 11 lorsque cette dernière est montée sur la poignée 2.

La tête 7 est globalement en forme de L. La grande barre verticale 70 reçoit, au voisinage de son extrémité supérieure libre 71, l'objectif 12 de la caméra.

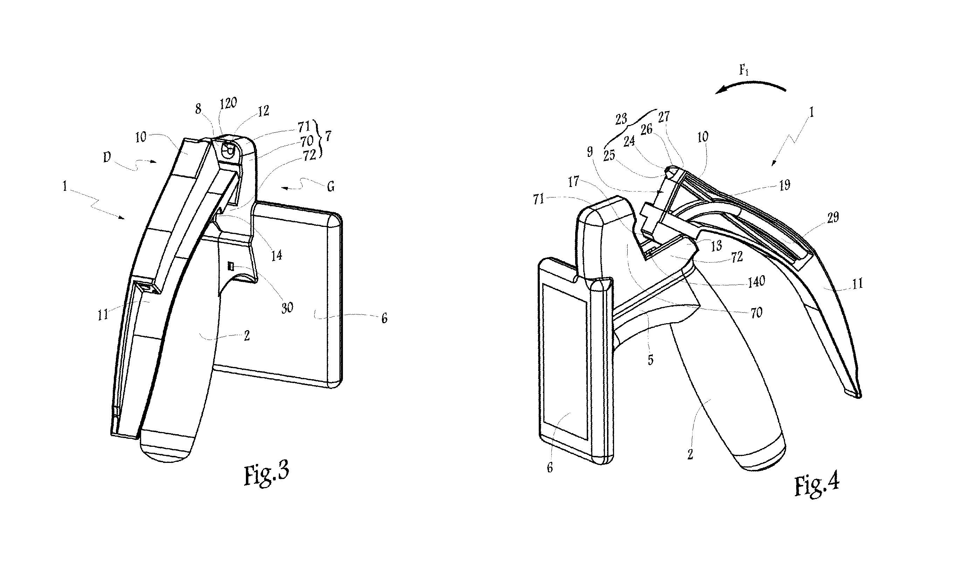

Cet objectif 12 est à mise au point automatique, c'est-à-dire autofocus. En variante, la mise au point est manuelle. Il occupe le fond d'un logement 120 de forme prédéterminée. Ce fond 130 est légèrement bombé, ce qui correspond à la courbure de la lentille de l'objectif de la caméra. Le logement 120 est en forme, globalement, de coin tronqué dont la base 121 est ouverte et s'évase sur l'extérieur. Dans un mode de réalisation non illustré, l'objectif forme lui-même le logement. Pour cela, il est en forme de coin tronqué. Les faces de ce logement 120 sont asymétriques. La face 122, orientée vers le haut aux figures 3, 5 et 6, est à courbure sphérique ou, en variante non illustrée, cylindrique. La courbure de la face 122 est une sphère centrée sur un axe de rotation de la lame 11, matérialisé par une tige 15. La face 122 est de dimensions différentes des deux faces latérales123, 124, planes, du logement 120 qui sont orientées vers la droite D et vers la gauche G du laryngoscope en regardant la figure 3. La face 125, orientée vers le bas aux figures 3, 5 et 6, est, à courbure conique.

En d'autres termes, les faces 123, 124 assurent la jonction entre une face conique 125 et une face sphérique 122.

La petite branche horizontale 72 de la tête 7 en forme de L, reçoit un dispositif de montage de la lame 11 sur la poignée 2. Ce dispositif est connu en soi et équipe certains laryngoscopes du marché. Ce dispositif de montage comprend deux pattes 13, 14, parallèles, reliées, au niveau de leurs bords libres, par une tige 15. Cette tige 15 définit un axe géométrique de rotation pour la lame 11 de laryngoscope. Le talon 10 de la lame 11 a une forme rectangulaire, adaptée pour être inséré dans le logement, de forme complémentaire, défini par les pattes 13, 14 de la tête 7.

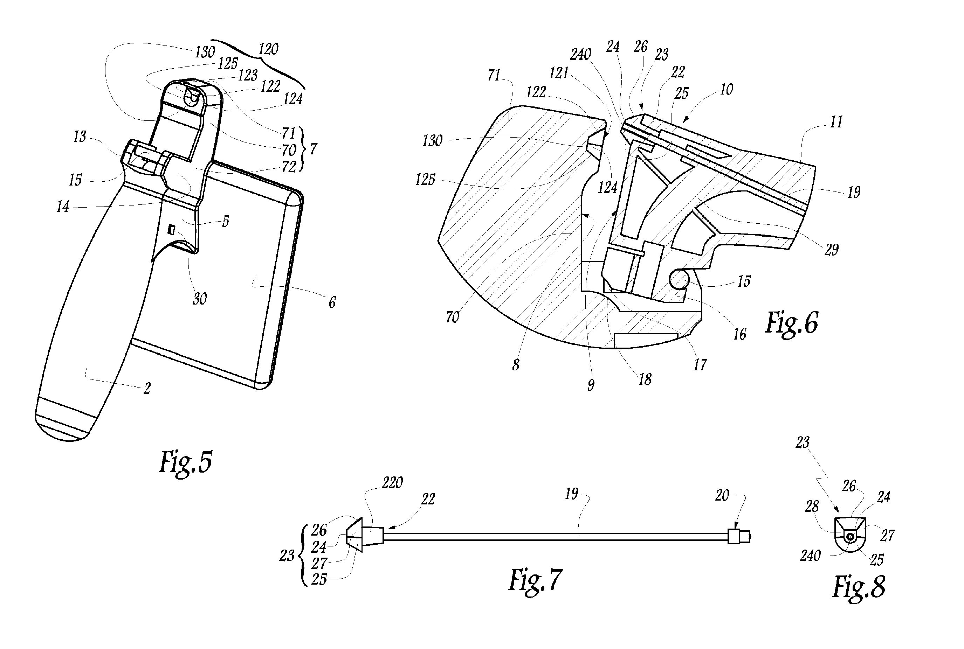

En l'espèce, le talon 10 de la lame 11 est équipé, en partie inférieure, d'un organe 16, en forme de crochet, adapté pour être en prise avec la tige 15 sur environ 2/3 de la circonférence de celle-ci et sur toute la longueur de la tige 15. Par un mouvement de la lame, réalisé selon une trajectoire en arc de cercle et effectué dans le sens inverse des aiguilles d'une montre en regardant la figure 6, on amène la face 9 située à l'arrière du talon 10 de la lame en appui contre la face 8 de la tête 7. Ce mouvement, illustré par la flèche F₁ à la figure 4, est effectué après que le crochet 16 soit mis en prise avec la tige 15.

Le mouvement illustré par la flèche F₁ permet de mettre la lame 11 en position d'utilisation sur la poignée 2, le laryngoscope étant alors prêt à être utilisé sur un patient.

Le crochet 16 est d'une forme et de dimensions lui permettant d'occuper tout l'espace disponible entre les pattes 13, 14 lorsque la lame 11 est montée sur la poignée 2. De manière connue, le crochet 16 comprend, au niveau du talon de la lame, deux lamelles 160, 161. Ces lamelles sont pourvues, chacune, d'un ergot 162, 163, orienté en direction des pattes 13, 14 lorsque la lame est en position sur la poignée 2. Les pattes 13, 14 comportent chacune une rainure. En l'espèce, la rainure 140 est visible à la figure 4. Lorsque la lame est en configuration d'utilisation, les lamelles 160, 161 sont insérés, en force, entre les pattes 13, 14 et les ergots 162, 163 sont logés dans les rainures. Lors d'un mouvement inverse au mouvement de mise en place de la lame, c'est-à-dire lors d'un mouvement visant à désolidariser la lame 11 et la poignée 2, la nature et la géométrie des ergots impliquent une rupture de ces derniers contre l'arête des rainures. De ce fait, si après avoir ôté la lame 11, on la remet en position sur la poignée 2, il n'est plus possible d'obtenir un blocage en configuration d'utilisation. On garantit ainsi que la lame est à usage unique.

Le crochet 16 est muni, sur au moins un côté et en partie basse, d'une encoche 17, visible à la figure 6. Cette encoche 17 permet la mise en place seulement de la lame 11 sur la poignée 2. Lorsque la lame 11 est en configuration de service, l'encoche 17 définit un logement de réception d'un relief 18 situé sur la barre horizontale 72 de la tête 7, au niveau de la zone de liaison avec l'autre barre 70.

En d'autres termes, le dispositif constitué par le relief 18 et l'encoche 17 forme un organe détrompeur évitant le montage d'une lame non dédiée sur la poignée 2. Il n'est donc pas possible de monter n'importe quelle lame sur une poignée 2, l'organe détrompeur n'autorisant le montage d'une lame 11 que sur une poignée du type de la poignée 2.

En revanche, il est possible de monter une lame du type de la lame 11 sur une poignée d'un autre laryngoscope que celui objet de l'invention, pour autant que le dispositif de connexion soit le même, c'est-à-dire comprenant un crochet 16 équipé de lamelles 160, 161, des pattes 13, 14 avec des rainures formant logement pour des ergots situés sur les lamelles 160, 161 et une tige 15.

Un guide d'images, en l'occurrence une fibre optique 19, est inséré, de manière définitive ou amovible, dans la lame 11. Avantageusement, la fibre optique 19 forme un dispositif optique passif solidaire de la lame 11. Cette fibre optique 19, connue en soi, est disposée selon une direction parallèle à un axe principal A-A' de la lame 11. Une extrémité libre 20 de la fibre optique 19 est équipée d'au moins une lentille optique. Cette extrémité, en position d'utilisation du laryngoscope, est située sur la face supérieure 21 de la lame 11, de manière à effectuer le guidage des images à partir de l'extrémité de la lame 11, lorsque le laryngoscope est utilisé. Cette extrémité 20 peut être équipée de filtres ou d'autres accessoires optiques.

L'extrémité opposée 22 de la fibre optique 19 est munie d'un moyen de connexion 23. L'extrémité 22 est configurée en cylindre pourvu d'un méplat 220. Ce méplat permet de guider et d'orienter le montage du moyen de connexion 23 de forme complémentaire. En d'autres termes, il s'agit d'un organe détrompeur adapté pour sécuriser le montage du moyen 23. En variante, l'extrémité 22 peut être équipée d'un organe détrompeur différent.

Le moyen de connexion est configuré, globalement, en forme de polyèdre 23 à sommet tronqué 24 et à faces asymétriques. Ce polyèdre 23 est de forme complémentaire à celle du logement 120. Ainsi, une face 25 est convexe à courbure conique, une face 26 est à courbure sphérique. Ces faces ont des dimensions différentes des faces latérales 27, 28, planes, qui les relient.

Le polyèdre 23 est monté de manière définitive ou amovible sur l'extrémité 22. La fibre 19 est insérée dans le polyèdre 23, à partir de la grande base de celle-ci, de sorte que l'extrémité 22 débouche, en position centrale, au sommet 24 du polyèdre 23. Le sommet 24 reçoit en position centrale une lentille optique 240 montée à l'extrémité de la fibre optique 19.

Comme représentée à la figure 4, lors du mouvement de mise en position d'utilisation de la lame 11 sur la poignée 2, après accrochage du crochet 16 sur la tige 15, il suffit de faire pivoter vers le haut, selon la flèche F₁, la lame 11 pour, d'une part, assurer un montage efficace et amovible de celle-ci sur la poignée, par clipsage, et d'autre part, réaliser un rapprochement entre la fibre optique 19 et l'objectif 12.

Ainsi, on a un premier moyen de connexion, comprenant le polyèdre 23 et le logement 120, adapté pour faire passer l'extrémité 22 pourvue de la lentille 240 d'une position où elle est éloignée de l'objectif 12 à une position où la lentille 240 est à proximité de l'objectif 12 et alignée avec ce dernier, leurs axes optiques respectifs étant confondus. Un deuxième moyen de connexion, comportant le crochet 16 les lamelles 160, 161, les rainures 140, les ergots et la tige 15, permet de faire passer la lame 11 d'une position dite de repos où elle n'est pas bloquée sur la poignée à une position où la lame est bloquée en configuration d'utilisation sur la poignée. Le mouvement, selon F₁, de la lame 11 entraîne en fin de course, l'alignement entre la lentille 240 et l'objectif 12.

En d'autres termes, un seul mouvement, selon une trajectoire en arc de cercle, permet la connexion des différents éléments du laryngoscope.

A l'issue du mouvement, le crochet 16 est clipsé sur la tige 15 et l'encoche 17 coopère avec le relief 18. Les dimensions des organes de connexion portés par la lame 11 et la tête 7 sont adaptées pour que la lame 11 soit maintenue en position tout en autorisant un retrait aisé de la lame 11, par un mouvement de rotation inverse, selon la flèche F₂ à la figure 2.

Lorsque la lame est en position sur la poignée, l'objectif 12 est maintenu aligné avec le sommet 24, c'est-à-dire avec la lentille 240 équipant l'extrémité 22 de la fibre optique 19. L'objectif 12 et la lentille 240, sont ainsi en face l'un de l'autre, avec un jeu minimal préservant l'absence de contact, et donc de frottement, entre ces deux éléments optiques.

Les formes complémentaires du logement 120 et du polyèdre 23 assurent, d'une part, un maintien, un guidage et un centrage de la lentille 240 de la fibre optique 19 et de l'objectif 12 de la caméra, de manière à éviter toutes distorsions des images et/ou tout parasitage par de la lumière externe.

La face 26 du polyèdre 23 et la face 122 du logement 120 sont les dernières parties du dispositif de connexion à entrer en contact l'une avec l'autre. Leurs dimensions et leurs formes facilitent l'insertion du polyèdre 23 dans le logement 120 en limitant tout frottement, et donc toute usure, entre ces deux éléments 23, 120.

Le centrage du polyèdre 23 dans son logement 120 est réalisé par la coopération, respectivement, de la face 25 du polyèdre 23 avec la face 125 du logement 120. La face 27 coopère avec la face 123 du logement, la face 28 avec la face 124 du logement.

Il est à noter que, lorsque les faces du logement 120 et du polyèdre 23 sont en contact, leurs formes induisent un blocage en position permettant d'assurer une stabilité optique entre l'objectif 12 et la lentille 240. Ainsi, on minimise les risques d'usure par frottement entre le logement 120 et l'extrémité 23 d'une lame, tout en ayant une qualité optimale de transmission d'images. En effet, une lumière parasite ne peut pas pénétrer entre l'objectif 12 et la lentille 240 du polyèdre 23 lorsque la connexion entre la lame 11 et la poignée 2 est effectuée.

On réalise une lame 11 à faible coût, puisqu'on utilise une fibre optique 19, à usage unique, insérée dans une lame 11, elle-même à usage unique, ces deux éléments, sans électronique et sans pièce complexe, sont de plus aisés à fabriquer de manière propre ou stérile. La caméra et son objectif sont réutilisables, puisque solidaires de la poignée 2. Leur position sur la poignée autorise un nettoyage aisé ce qui permet d'utiliser des matériaux courants. La configuration des systèmes de connexion permet aisément de nettoyer l'ensemble, notamment la poignée 2, pour assurer une hygiène optimale.

Par ailleurs, la lame 11 peut être fixée, si besoin, sur un autre type de poignée, par exemple celle d'un autre laryngoscope puisque le système de fixation par crochet est un système couramment employé. Une telle lame peut être équipée d'un ensemble fibre optique 19 et extrémités 20, 22 amovibles. Dans ce cas, la mise en place du guide d'images est réalisée, à la demande, par l'utilisateur.

La position, telle que représentée, de l'écran 6 par rapport à la lame, c'est-à-dire situé dans le prolongement de la lame 11, permet d'avoir dans le champ de vision de l'utilisateur une vision « en réelle » et sur l'écran 6, de la cavité buccale et de la trachée du patient. Pour cela, la lame 11 est avantageusement équipée d'un dispositif d'éclairage 29 dont la connexion est faite au niveau du talon 10 de la lame 11, lorsqu'elle est en position sur la poignée 2.

Une telle poignée 2 peut également être équipée d'un dispositif de transmission sans fil, par exemple de type WIFI, assurant la transmission des données en direction d'un ordinateur distant afin d'assurer un double contrôle. De même une connexion 30, visible aux figures 1 et 5, permet une connexion avec tout dispositif connu en soi muni d'un guide d'images et d'un guide de lumière.

Dans un autre mode de réalisation non représenté, la forme de l'organe de connexion est différente. Par exemple, il peut s'agir d'une tête de forme carrée et non en L. Dans ce cas, on effectue la mise en place, non par rotation de la lame, mais par translation, la lame glissant entre deux rails ménagés sur la poignée.

## Revendications

1. Laryngoscope comportant :
- une poignée (2) formant un logement pour une caméra dont l'objectif (12) est affleurant à une partie (8) de la poignée (2),
- une lame de laryngoscope (11) montée de manière amovible sur la poignée (2),
- un guide d'images (19) monté sur la lame (11) et adapté pour transmettre les images entre une extrémité (20) de la lame (11) et la caméra, **caractérisé en ce qu'**il comprend également :
- un premier moyen de connexion (23, 120), entre l'objectif (12) de la caméra et une lentille optique (240) montée à une extrémité (22) du guide d'images (19), adapté pour faire passer la lentille optique (240) d'une position où cette lentille (240) est éloignée de l'objectif (12) de la caméra à une position où la lentille optique (240) est à proximité et alignée avec l'objectif (12) de la caméra, leurs axes optiques étant confondus et situés dans le prolongement d'un axe principal (A-A') de la lame (11),
- un deuxième moyen de connexion (15, 16) entre la lame (11) et la poignée (2), adapté pour faire passer la lame (11) d'une position où elle n'est pas bloquée en configuration d'utilisation, sur la poignée (2), à une position où la lame (11) est bloquée en configuration d'utilisation sur la poignée et **en ce que** le passage (F₁) de la lame (11) à la position où elle est en configuration d'utilisation entraîne le passage (F₁) de l'extrémité (22) du guide d'images (19) équipée de la lentille optique (240) dans la position où cette lentille (240) est à proximité et alignée avec l'objectif (12) de la caméra.

2. Laryngoscope selon la revendication 1, **caractérisé en ce que** le moyen de connexion comprend un logement (20), ménagé sur la poignée (2), et configuré en forme de coin tronqué à faces asymétriques (122, 123, 124, 125).

3. Laryngoscope selon la revendication 1, **caractérisé en ce que** le moyen de connexion comprend un organe configuré en forme de polyèdre (23) tronqué, à faces asymétriques (25, 26, 27, 28), et disposé à une extrémité (22) du guide d'images (19).

4. Laryngoscope selon les revendications 2 et 3, **caractérisé en ce que** le logement (120) et le polyèdre (23), de formes complémentaires, sont respectivement positionnés pour que leurs parties tronquées (130, 24) reçoivent, d'une part (130), l'objectif (12) de la caméra et, d'autre part (24), la lentille optique (240) de l'extrémité (22) du guide d'images (19).

5. Laryngoscope selon l'une des revendications 2 à 4, **caractérisé en ce qu'**une des faces (122) du logement (120) et une des faces (26) du polyèdre (23) sont à courbure sphérique.

6. Laryngoscope selon l'une des revendications précédentes, **caractérisé en ce que** le passage (F₁) de la lame (11) à une configuration d'utilisation sur la poignée (2) et le passage (F₁) de l'extrémité (22) du guide d'images (19) dans la position où la lentille optique (240) est à proximité de l'objectif (12) de la caméra suivent des trajectoires en arc de cercle.

7. Laryngoscope selon l'une des revendications précédentes, **caractérisé en ce que** la poignée (2) est équipée d'un écran de visualisation (6).

8. Laryngoscope selon la revendication 7, **caractérisé en ce que** l'écran de visualisation (6) est positionné selon l'axe principal (A-A') de la lame (11).

9. Laryngoscope selon l'une des revendications précédentes, **caractérisé en ce que** l'écran est déporté, la liaison entre l'écran et la poignée du laryngoscope étant filaire ou non.

10. Laryngoscope selon l'une des revendications précédentes, **caractérisé en ce que** la lame (11) est pourvue, à son extrémité (10) adaptée pour être montée sur la poignée, d'un organe (18, 17) autorisant le montage seulement d'une lame de ce type sur cette poignée (2).

## Revendications modifiées

### Revendications modifiées conformément à la règle 137(2) CBE.

**1.** Laryngoscope comportant :
- une poignée (2) formant un logement pour une caméra dont l'objectif (12) est affleurant à une partie (8) de la poignée (2),
- une lame de laryngoscope (11) montée de manière amovible sur la poignée (2),
- un guide d'images (19) monté sur la lame (11) et adapté pour transmettre les images entre une extrémité (20) de la lame (11) et la caméra,
- un premier moyen de connexion (23, 120), entre l'objectif (12) de la caméra et une lentille optique (240) montée à une extrémité (22) du guide d'images (19), adapté pour faire passer la lentille optique (240) d'une position où cette lentille (240) est éloignée de l'objectif (12) de la caméra à une position où la lentille optique (240) montée à l'extrémité du guide d'images (19) est à proximité et alignée avec l'objectif (12) de la caméra, **caractérisé en ce que**, dans cette position, les axes optiques de la lentille (240) du guide d'images (19) et de l'objectif (12) de la caméra sont confondus et situés dans le prolongement d'un axe principal (A-A') de la lame (11), et **en ce qu'**il comprend :
- un deuxième moyen de connexion (15, 16) entre la lame (11) et la poignée (2), adapté pour faire passer la lame (11) d'une position où elle n'est pas bloquée en configuration d'utilisation, sur la poignée (2), à une position où la lame (11) est bloquée en configuration d'utilisation sur la poignée et **en ce que** le passage (F₁) de la lame (11) à la position où elle est en configuration d'utilisation entraîne le passage (F₁) de l'extrémité (22) du guide d'images (19) équipée de la lentille optique (240) dans la position où cette lentille (240) est à proximité et alignée avec l'objectif (12) de la caméra.

**2.** Laryngoscope selon la revendication 1, **caractérisé en ce que** le moyen de connexion comprend un logement (20), ménagé sur la poignée (2), et configuré en forme de coin tronqué à faces asymétriques (122, 123, 124, 125).

**3.** Laryngoscope selon la revendication 1, **caractérisé en ce que** le moyen de connexion comprend un organe configuré en forme de polyèdre (23) tronqué, à faces asymétriques (25, 26, 27, 28), et disposé à une extrémité (22) du guide d'images (19).
